# EUROPÄISCHE PATENTSCHRIFT

(11) **EP 0 709 088 B1**
(45) Veröffentlichungstag und Bekanntmachung des Hinweises auf die Patenterteilung: **28.05.1997**
(21) Anmeldenummer: 95110485.0
(22) Anmeldetag: 05.07.1995
(51) Int. Cl.: A61K 9/70, A61K 31/485, A61K 47/32

(54) **Transdermales therapeutisches System zur Applikation von Naloxon**
Transdermal therapeutic system for application of naloxone
Système d'administration transdermique de naloxone

(30) Priorität: 07.07.1994 DE 4423850
(43) Veröffentlichungstag der Anmeldung: 01.05.1996
(73) Patentinhaber: LABTEC GESELLSCHAFT FüR TECHNOLOGISCHE FORSCHUNG UND ENTWICKLUNG MBH, D-40764 Langenfeld (DE)
(72) Erfinder: Cordes, Günter, Dr., D-42799 Leichlingen (DE); Vollmer, Ulrike, Dr., D-41542 Dormagen (DE)

(56) Entgegenhaltungen:
- EP-A- 0 178 140
- WO-A-90/04965
- DE-A- 3 614 843
- PHARMACEUTICAL RESEARCH, Bd. 7, Nr. 7, 1990 Seiten 712-718, B.J. AUNGST ET AL. 'Contributions of drug solubilisation, partitioning, barrier disruption, and solvent permeation to the enhancement of skin permeation of various compounds with fatty acids and amines'

## Beschreibung

Die vorliegende Erfindung betrifft ein auf der Haut anzubringendes Pflaster, auch transdermales therapeutisches System (TTS) genannt, zur Verabreichung der Substanz Naloxon = 1-N-allyl-7,8-dihydro-14-hydroxynormorphinon an den Menschen. Wird im folgenden der Ausdruck Naloxon verwendet, so ist darunter Base, Hydrochlorid oder Hydrochlorid mit Kristallwasser zu verstehen.

Naloxon wird bereits in parenteraler Form angewendet, z. B. bei postoperativer Atemlähmung oder bei der Therapie Suchtkranker. Es ist allerdings therapeutisch zweckmäßig, nach der Injektion im akuten Erkrankungszustand über eine längere Zeit eine niedrige Dosis Naloxon dem Erkrankten zuzuführen. Eine orale Gabe kommt wegen der schlechten Steuerbarkeit nicht in Frage, die Substanz wird in der Leber stark metabolisiert (First Pass-Effekt). Mit einem TTS jedoch wäre die Behandlung möglich, falls es gelingt, die erforderliche Dosis Naloxon kontrolliert durch die Haut in den systemischen Kreislauf zu bringen.

In vielen Fällen ist ein Problem bei der Verabreichung von Arzneistoffen aus TTS die Erzielung eines ausreichend hohen Fluxes durch die Haut und damit die Erzielung genügend hoher Blutspiegel. Man begegnet diesem Problem entweder dadurch, daß man geeignete Enhancer hinzufügt, die die Durchlässigkeit der Haut für den Wirkstoff verbessern und/oder man wählt eine möglichst hohe Konzentration der/des Wirkstoffe(s) in der Matrix-Klebermasse. Durch die hohe Konzentration soll eine hohe thermodynamische Aktivität erzielt werden und somit eine höhere Permeation durch die Haut ermöglicht werden. Schließlich kann man auch bestimmte Salze oder Ester von Wirkstoffen einsetzen, wenn dadurch eine bessere Hautpermeation gegeben ist.

Aus WO-A-90/04 965 Beispiel 2 (Seiten 10 bis 11) ist ein transdermales therapeutisches System bekannt, das Naloxon-Base in einem Zellulose-Gel zwischen Trägerfolie und Haftkleber vorsieht. Ferner beschreibt DE-A-3 614 843 in Beispiel 1 ein ähnliches Arzneimittelreservoir mit einem Gehalt an Naloxonhydrochlorid in einem Zellulose-Gel, wobei das TTS zusätzlich noch ein Penetrationsbeschleuniger-Reservoir vorsieht.

Ferner findet man in EP-A2-0 178 140 den Hinweis, daß bei Einsatz von Naloxonhydrochlorid die Permeationsrate durch die Haut zu gering ist und daß der Zusatz von Enhancern entweder keine Erhöhung ergibt oder aber die Hautverträglichkeit in unzumutbarer Weise verschlechtert. Man hat dann den Weg beschritten, nicht das Hydrochlorid einzusetzen, sondern die freie Base. Allerdings war es nicht möglich, ohne Enhancer auszukommen, um den erforderlichen Flux durch die Haut zu erzielen. Der verwendete Enhancer beeinflußte jedoch die Klebeeigenschaften der zur Herstellung der TTS verwendeten Kleber negativ, was nur durch einen hohen technischen Aufwand, nämlich das Aufbringen einer zusätzlichen, an den Rändern des Reservoirs überstehenden Klebeschicht zu beheben war. Durch den dadurch bedingten komplizierten Aufbau werden die Herstellkosten des Produktes in die Höhe getrieben.

Erfindungsgemäß wird ein transdermales Naloxon enthaltendes therapeutisches System vorgesehen, bestehend aus einer Abziehfolie, einem Haftkleber auf Acrylat-Basis mit eingearbeitetem Wirkstoff und einer Trägerfolie, wobei als Wirkstoff Naloxonhydrochlorid, Naloxonhydrochloriddihydrat oder ein anderes Naloxonsalz in einer Menge von 0,2 bis 2,0 mg/cm² sowie soviel eines Alkalihydroxides eingearbeitet wurde, daß der pH-Wert der Polymerlösung während der Herstellung zwischen 7,2 und 8,7 liegt.

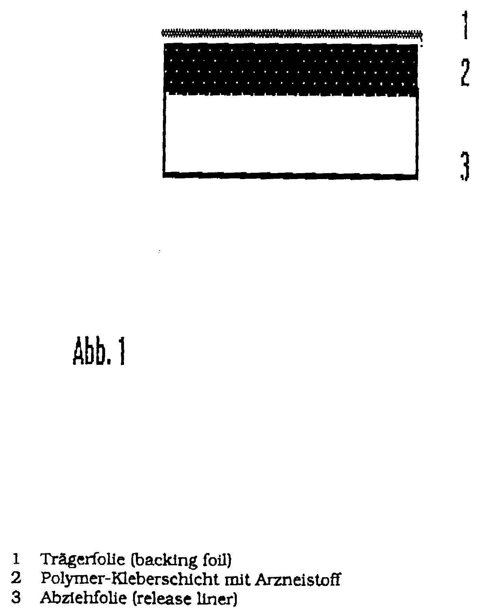

Das Pflaster besteht also aus einer Trägerfolie (1, Abb. 1), einem Haftkleber mit der Wirksubstanz (2, Abb. 1) und einer Abziehfolie (3, Abb. 1).

Überraschend wurde in dieser Erfindung entdeckt, daß ein TTS mit Naloxonhydrochlorid hergestellt werden kann, das eine ausreichende Menge an Wirkstoff durch Haut freisetzt, ohne daß ein Enhancer dem Produkt zugesetzt werden muß. Die Freisetzung, auch Flux bezeichnet, betrug, wie später ausführlich dargestellt, 10,9 µg/cm²/h durch hairless mouse skin. Im Vergleich dazu haben Aungst et al. in Pharm. Res., 7 (1990) 712 - 718 bei Verwendung einer enhancerhaltigen Lösung von Naloxon-Base nur 3,6 µg/cm²/h gefunden. Daraus geht hervor, daß das hier beschriebene erfindungsgemäße Produkt eine hohe Wirkstoff-Freigaberate an der Haut besitzt, obwohl keine Enhancer eingesetzt sind und das Produkt einen einfachen technologischen Aufbau besitzt (Abb. 1).

Bei dem erfindungsgemäßen transdermalen therapeutischen System kann der Haftkleber auf Acrylat-Basis durch radikalische Copolymerisation von 2-Ethylhexylacrylat, Vinylacetat, Hydroxyethylacrylat oder Mischungen daraus erhalten worden sein.

Das erfindungsgemäße transdermale therapeutische System kann durch eine Menge von bis zu 5 % Vernetzer gekennzeichnet sein.

So kann der erfindungsgemäße Haftkleber aus 50 bis 75 % und vorzugsweise 60 bis 70 % 2-Ethylhexylacrylat, 20 bis 30 % Vinylacetat, 3 bis 9 % Hydroxyethylacrylat, < als 1 % Glycidylmethacrylat und < als 1 % Polybutyltitanat gewonnen worden sein.

Das erfindungsgemäße transdermale System kann eine Abziehfolie aus silikonisiertem Polyethylenterephthalat und eine Trägerfolie aus Polyethylenterephthalat aufweisen.

Als Alkalihydroxid können KOH oder NaOH eingesetzt werden.

### Beispiele

### Beispiel 1:

Zur Herstellung von 200 Naloxon HCl Pflastern von je 10 cm² Fläche werden
- 2,635 g: Naloxon HCl x 2H₂O mikronisiert mit
- 42 ml: Ethanol und
- 23,7 g: 0,5 N ethanol. KOH
- 10 min im: Ultraschallbad behandelt. Danach werden
- 86,8 g: Durotak^{R} 280-2516 (handelsübliche 40,7 %-igen Lösung eines Acrylathaftklebers)
hinzugefügt und 2 Std. mit einem Magnetrührer gerührt.

Danach wird die Lösung auf eine handelsübliche silikonisierte Polyethylenterephthalatfolie von ca. 100 µ Dicke ausgestrichen und eine Stunde bei 40 °C und danach 18 Stunden bei 25 °C getrocknet. Anschließend wird eine nicht silikonisierte Polyethylenterephthalatfolie von ca 15 µm Dicke aufkaschiert. Es werden Pflaster von 10 cm² Fläche ausgestanzt und in üblicher Weise verpackt. Der theoretische Gehalt eines TTS beträgt 4,0 mg Naloxon HCl x 2 H₂O entsprechend 3,274 mg Naloxon Base.

Untersuchung der Pflaster auf Wirkstoff-Freisetzung:

Die Pflaster werden nach USP XXII in der Apparatur "Paddle over Disk" freigesetzt. Als Analysenmethode wird eine modifizierte HPLC-Methode angewendet; vgl. Tawakkol et al. in J. Liq. Chromat., 6 (1983). Die Freisetzung beträgt
8 % nach 15 min,
25 % nach 2 Stunden und
50 % nach 8 Stunden.

Untersuchung der Pflaster auf Wirkstoff-Freisetzung:

Die Pflaster werden in der Franz-Zelle (4) unter Verwendung von hairless mouse skin auf die Naloxon-Permeation untersucht; vgl. Franz in J. Invest. Dermatol., 64 (1975) 191 - 195. Die Analysenmethode erfolgt analog der Freisetzungsbestimmung. Die Untersuchung ergab einen Steady State Flux von 10,9 µg/cm²/h über die ersten 6 Stunden mit einer lag-time unter einer Stunde, was für die Therapie mit diesem Produkt vorteilhaft ist.

## Patentansprüche

1. Transdermales Naloxon enthaltendes therapeutisches System, bestehend aus einer Abziehfolie, einem Haftkleber und einer Trägerfolie, dadurch **gekennzeichnet**, daß in einen Haftkleber auf Acrylat-Basis Naloxonhydrochlorid, Naloxonhydrochlorid-Dihydrat oder ein anderes Naloxonsalz in einer Menge von 0,2 bis 2,0 mg/cm² sowie soviel eines Alkalihydroxides eingearbeitet wurde, daß der pH-Wert der Polymerlösung während der Herstellung zwischen 7,2 und 8,7 liegt.

2. Transdermales therapeutisches System nach Anspruch 1, dadurch **gekennzeichnet**, daß der Haftkleber auf Acrylat-Basis erhalten worden ist durch radikalische Copolymerisation von 2-Ethylhexylacrylat, Vinylacetat, Hydroxyethylacrylat oder Mischungen daraus.

3. Transdermales therapeutisches System nach Anspruch 1 oder 2, **gekennzeichnet** durch eine Menge von bis zu 5 % Vernetzer.

4. Transdermales therapeutisches System nach einem der vorhergehenden Ansprüche, dadurch **gekennzeichnet**, daß der Haftkleber gemäß Anspruch 2 gewonnen worden ist aus 50 bis 75 % und vorzugsweise 60 bis 70 % 2-Ethylhexylacrylat, 20 bis 30 % Vinylacetat, 3 bis 9 % Hydroxyethylacrylat, < 1 % Glycidylmethacrylat und < 1 % Polybutyltitanat.

5. Transdermales therapeutisches System nach einem der vorhergehenden Ansprüche, **gekennzeichnet** durch eine Abziehfolie aus silikonisiertem Polyethylenterephthalat und eine Trägerfolie aus Polyethylenterephthalat.

6. Transdermales therapeutisches System nach einem der vorhergehenden Ansprüche, **gekennzeichnet** durch KOH oder NaOH als Alkalihydroxid.

## Claims

1. Transdermal Naloxon-containing therapeutic system comprising a release liner, an adhesive, and a backing foil whereby Naloxon hydrochloride, Naloxon hydrochloride dihydrate, or some other Naloxon salt is incorporated into an acrylic-based adhesive at the rate of 0.2 to 2.0 mg/cm² along with as much alkali hydroxide as is necessary to adjust the pH of the polymer solution during the production process to between 7.2 and 8.7.

2. Transdermal therapeutic system as per Claim 1, characterised by the fact that the acrylic-based adhesive is obtained through the radical copolymerisation of 2-ethylhexyl acrylate, vinyl acetate, hydroxyethyl acrylate, or of mixtures of the same.

3. Transdermal therapeutic system as per Claims 1 or 2, characterised by the fact that it contains up to 5 % cross-linking agent.

4. Transdermal therapeutic system as per one of the above claims, characterised by the fact that the adhesive as per Claim 2 is obtained from 50 - 75 % but preferably 60 to 70 % 2-ethylhexyl acrylate, 20 to 30 % vinyl acetate, 3 to 9 % hydroxyethyl acrylate, < 1 % glycidyl methacrylate and < 1 % polybutyl titanate.

5. Transdermal therapeutic system as per one of the above claims, characterised by a release liner of siliconised polyethylene terephthalate, and a backing foil of polyethylene terephthalate.

6. Transdermal therapeutic system characterised by the use of KOH or NaOH as the alkali hydroxide.

## Revendications

1. Système thérapeutique transdermique contenant du Naloxon, composé d'un film à retirer, d'une colle d'adhésion et d'un film support caractérisé en ce que du Naloxonhydrochlorure, du dihydrate de Naloxonhydrochlorure ou un autre sel Naloxon en une quantité de 0,2 à 2,0 mg/cm² ainsi que d'autant d'un hydroxyde alcalin ont été intégré dans une colle d'adhésion sur la base d'un acrylate pour que la valeur pH de la solution polymère se situe entre 7,2 et 8,7 pendant la production.

2. Système thérapeutique transdermique selon la revendication 1 caractérisé en ce que la colle d'adhésion a été obtenue sur la base d'un acrylate par copolymérisation radicale de 2-acrylate hexylique d'éthyle, acétate vinylique, acrylate d'hydroxyéthyle ou de mélanges de ces produits.

3. Système thérapeutique transdermique selon les revendications 1 et 2 caractérisé par une quantité de 5% de réticulant max.

4. Système thérapeutique transdermique selon l'une des revendications précédentes caractérisé en ce que la colle d'adhésion est obtenue conformément à la revendication 2 à partir de 50 à 75% et, de préférence, 60 à 70% de 2-acrylate hexylique d'éthyle, 20 à 30% d'acétate vinylique, 3 à 9% d'acrylate d'hydroxyéthyle, < de 1% de méthacrylate glycidilique et < de 1% de polybutènetitanate.

5. Système thérapeutique transdermique selon l'une des revendications précédentes caractérisé par un film à retirer en polyéthylène-téréphtalate traité au silicone et par un film support en polyéthylène-téréphtalate.

6. Système thérapeutique transdermique selon l'une des revendications précédentes caractérisé par KOH ou NaOH comme hydroxyde alcalin.
